# EUROPEAN PATENT SPECIFICATION

(11) **EP 2 545 856 B1**
(45) Date of publication and mention of the grant of the patent: **09.09.2015**
(21) Application number: 12187841.7
(22) Date of filing: 05.03.2009
(51) Int. Cl.: A61B 6/00, A61B 6/04, G05G 1/445, A61G 13/02, H01H 13/14, H01H 13/26, H01H 21/26

(54) **Foot-activated controller for medical system**
Fußbetätigte Steuerung für medizinisches System
Unité de commande activée par pédale pour système médical

(30) Priority: 13.03.2008 US 36135 P
(43) Date of publication of application: 16.01.2013
(62) Divisional of application: 09719409.6
(73) Proprietor: Liebel-Flarsheim Company LLC, Hazelwood MO 63042 (US)
(72) Inventor: Ellafrits, David J, Vicksburg, MI Michigan 49097 (US)
(74) Representative: Edson, Russell Gregory

(56) References cited:
- US-A- 5 883 615
- US-A- 6 051 797
- US-B1- 6 234 672

## Description

### FIELD OF THE INVENTION

The present invention generally relates to the field of medical systems that utilize at least one medical device and, more particularly, to foot-activated controllers for communicating with such a medical device.

### BACKGROUND

Medical imaging systems exist that utilize an adjustable patient table and appropriate imaging equipment. One such imaging system is commonly referred to as a "urology table." Urology tables are used to perform various urology procedures. It is common for these types of medical imaging systems to utilize foot-activated controllers for communicating with the movable patient table and the imaging equipment. One foot-activated controller is typically provided for communicating with the movable patient table, while a separate foot-activated controller is typically provided for communicating with the imaging equipment.

Known foot-activated controllers for the patient table incorporate a number of pedals or switches for controlling the position of the patient table. Patient tables for urology applications typically are movable in each of a vertical dimension, as well as longitudinal and lateral dimensions within a reference plane that at least generally coincides with a supporting surface of the patient table. These tables may also be tilted about a horizontal axis (e.g., to raise the patient's head and simultaneously lower the patient's feet; to lower the patient's head and simultaneously raise the patient's feet). Known foot-activated controllers for the imaging equipment incorporate a number of pedals or switches for controlling various aspects of the image acquisition function.

US-A-5883615 discloses a medical system from which the prior art features of the present invention may be derived.

### SUMMARY

According to the invention there is provided a medical system according to claim 1 appended hereto. Other features of the invention are claimed in sub-claims appended hereto.

The present invention includes a first medical device, a first controller, and a second controller. The first controller is operatively interconnected with the first medical device and is foot-activated (e.g., a foot-activated controller). The second controller is also operatively interconnected with this same first medical device and is foot-activated as well (e.g., another foot-activated controller). The first controller may provide a first set of functions for the first medical device, while the second controller may provide a second set of functions for this same first medical device. Each function in the first set of functions (associated with the first controller) may also be a function in the second set of functions (associated with the second controller). The number of functions in the second set of functions (associated with the second controller), may be greater than the number of functions in the first set of functions (associated with the first controller). Therefore, the first controller may be characterized as providing a subset of the functions provided by the second controller, where the entirety of the subset (the functions provided by the first controller) is contained within and is smaller than the set (the functions provided by the second controller).

A plurality of first actuators is incorporated by the first controller, where each of these first actuators is disposed at a common elevation and where each first actuator is able to communicate with the first medical device. A central, longitudinal axis bisects the first controller in a lateral dimension (e.g., the lateral dimension being a side-to-side dimension, for instance such that an operator's foot would move at least generally orthogonally to the lateral dimension to approach and use the first controller). Each first actuator that is disposed on a first side of this central, longitudinal axis is in a common first orientation, while each first actuator that is disposed on a second side of this central, longitudinal axis is in a common second orientation, where the first and second orientations are different (e.g., the first and second sides are opposite sides of this central, longitudinal reference axis). At least one first actuator is disposed on the first side of the central, longitudinal axis, and at least one first actuator is disposed on the second side of the central, longitudinal axis.

The present invention may be embodied by a medical system that includes imaging equipment, a table assembly that in turn includes a movable table and a table positioner that interacts with this table, and first and second controllers. The first and second controllers are each operatively interconnected with at least the imaging equipment, and furthermore are each foot-activated. The first controller may control a first number of functions of the imaging equipment, while the second controller may control a second number of functions of the imaging equipment, where the second number of functions is greater than the first number of functions. That is, the second controller controls more functions of the imaging equipment than does the first controller.

The medical system utilizes a second medical device. The first controller is operatively interconnected with this second medical device, but not the second controller. Although each of the first and second medical devices may be of any appropriate type, in one embodiment the first and second medical devices are imaging equipment and a table assembly (e.g., having a movable table), or vice versa.

The first controller may be programmable. Any appropriate way of programming the first controller may be utilized, for instance by using a laptop or other computer that is in communication with the first controller in any appropriate manner, for instance by including one or more appropriate communication ports on the first controller (e.g., a wireless communication port; a serial communication port). At least one actuator that is incorporated by the first controller may be assigned a function(s) by having the first controller be programmable. The first controller may be of any appropriate size, shape, configuration, and/or type. For example, in one embodiment, the first controller is of an at least generally V-shaped or boomerang-shaped configuration in a top view, with the opening of this configuration defining a forward end of the first controller.

At least two of the first actuators may be disposed on the first side of the central, longitudinal axis, and at least two of the first actuators may be disposed on the second side of the central, longitudinal axis. Each first actuator that is disposed on the first side of the central, longitudinal axis moves at least generally about a first reference axis, and each first actuator that is disposed on the second side of the central, longitudinal axis moves at least generally about a second reference axis, where an included angle between these first and second reference axes is obtuse (e.g., greater than 90° and less than 180°). A common number of first actuators may be disposed on each of the first and second sides of this central, longitudinal axis, where the first and second sides are a mirror image of each other in relation to an arrangement that includes at least one first actuator.

The first controller may include upper and lower sections. A plurality of first actuators may be incorporated on the lower section of this first controller. At least one second actuator may be incorporated on upper section of this first controller. In one embodiment, a single second actuator in the form of a four-position switch is incorporated by the upper section, and this four-position switch may be disposed on the noted central, longitudinal axis. In one embodiment, each first actuator that is incorporated on the lower section is designated for controlling an imaging function, and at least one actuator that is incorporated on the upper section is designated for controlling movement of a certain portion of the medical system (e.g., a table, part of the imaging equipment (e.g., the imaging chain)). One or more switches may be included on the upper section, for instance a mode switch (e.g., to change what portion of the medical system is moved by an actuation of the noted second actuator, for instance to change between the table assembly and the imaging chain), a save image switch, or both.

The medical system of the present invention may be utilized for any appropriate application, including any appropriate medical application (e.g., for performing one or more urology procedures). In one embodiment and where an imaging assembly is being utilized, the medical system may be characterized as a medical imaging system. Any appropriate imaging equipment may be utilized by the medical system, including without limitation one or more components for providing an imaging functionality such as x-ray, tomography, fluoroscopy, endoscopy, and any combination thereof.

Any table assembly that is incorporated by the medical system of the present invention may include a table that is movable in any appropriate manner and/or in any appropriate dimension or combination of dimensions. The structure that moves the table may be referred to as a table positioner. Any number of movement options of any appropriate type may be utilized by the noted table. The table may be moved in each of first and second directions within a reference plane that at least generally coincides with a supporting surface of the table. These two different directions may be orthogonal to each other - for instance one defining a longitudinal dimension or longitudinal axis (e.g., coinciding with a height dimension of a patient lying on the table, or coinciding with a dimension in which the patient's head and feet are spaced when lying on the table) and the other defining a lateral dimension or axis (e.g., coinciding with a dimension in which a patient's shoulders would be spaced if the patient were to lie on his/her back on the table in the above-noted manner). The longitudinal dimension or axis may coincide with the long axis of the supporting surface of the table, while the lateral dimension or axis may coincide with the short axis of the supporting surface of the table.

Another motion that any such table may undergo is in the vertical dimension - a motion that changes the elevation of the table (and including the entirety of its supporting surface). Yet another type of motion that may be utilized for this table is a movement at least generally about a first axis. This first axis is subject to a number of characterizations, which apply individually and in any appropriate combination. For instance, the first axis may be horizontally disposed, may extend in the lateral dimension, or both. In one embodiment, the lateral dimension of the supporting surface of the table is maintained parallel to horizontal. Movement of the table at least generally about the first axis may be characterized as a tilting of the table. The angle at which the table is disposed relative to horizontal (e.g., the angle between the longitudinal axis of the table and horizontal) may be referred to as a "tilt angle." Tilting of the table may be undertaken to raise the patient's head and simultaneously lower the patient's feet, may be undertaken to lower the patient's head and simultaneously raise the patient's feet, or both.

### BRIEF DESCRIPTION OF THE FIGURES

Figure 1 is a schematic of one embodiment of an imaging system that uses a foot-activated controller.
Figure 2 is a more detailed view (perspective) of the imaging system of Figure 1.
Figure 3 is a perspective view of one embodiment of a foot-activated controller that may be used by the imaging systems of Figures 1 and 2.
Figure 3A is a plan view of a rear panel of the foot-activated controller of Figure 3.
Figure 4 is one embodiment of a functional schematic that may be utilized by the foot-activated controller of Figure 3.
Figure 5 is one embodiment of a programming protocol that may be utilized by the foot-activated controller of Figure 3.
Figure 6 is one embodiment of an operations protocol that may be utilized by the foot-activated controller of Figure 3.
Figure 7 is a schematic of one embodiment of an imaging system that uses at least two foot-activated controllers.
Figure 8 is a perspective view of another embodiment of a foot-activated controller that may be used by the imaging systems of Figure 7.

### DETAILED DESCRIPTION

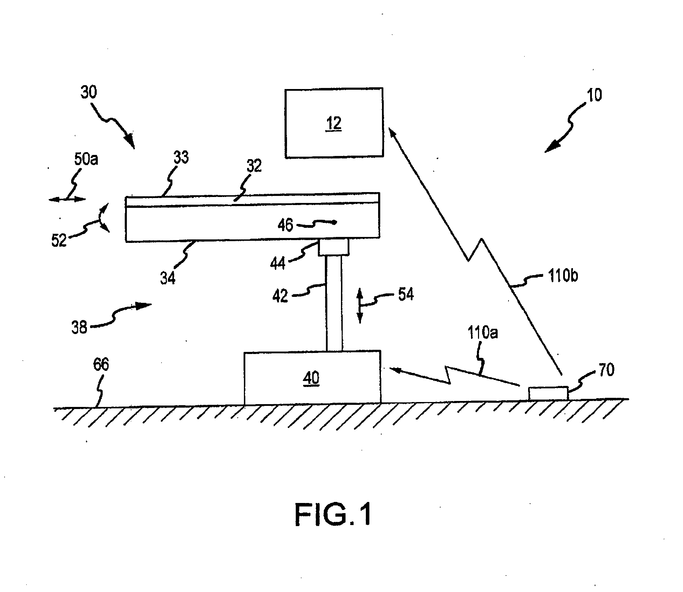

One embodiment of an imaging system is illustrated in Figure 1 and is identified by reference numeral 10. The imaging system 10 may be used for any appropriate application, including without limitation a medical application. Therefore, the imaging system 10 may be referred to as a medical imaging system 10.

The medical imaging system 10 includes an imaging assembly 12 and a table assembly 30, each of which may be of any appropriate size, shape, configuration, and/or type. The imaging assembly 12 may include any appropriate imaging equipment and any related components (e.g., for providing an x-ray functionality (e.g., acquiring an x-ray image), for providing a tomography functionality (e.g., acquiring a tomography image), for providing a fluoroscopy functionality (e.g., acquiring a fluoroscopy image), endoscopy, and any combination thereof). Although the medical imaging system 10 may be configured for any appropriate medical application, in one embodiment the medical imaging system 10 is adapted for performing/facilitating the performance of one or more urology procedures.

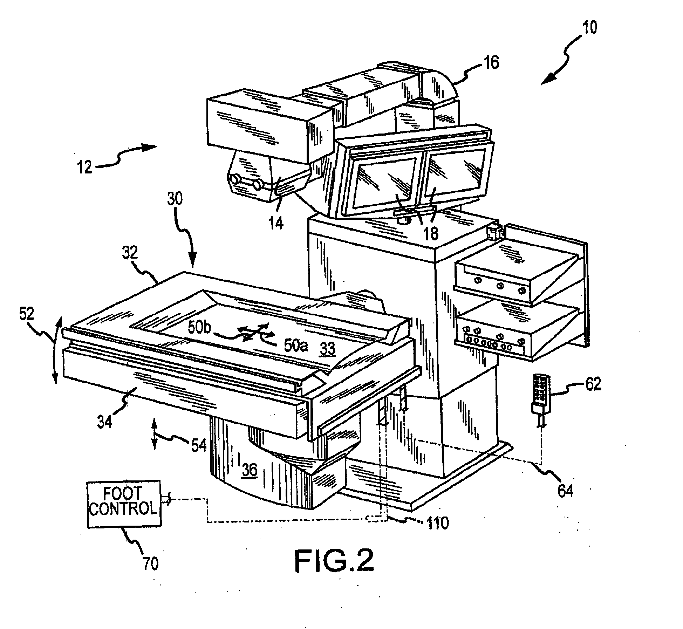

The table assembly 30 may include a table or a tabletop 32, a table tub 34, and a table positioner 38. The table 32 may be moved relative to the table tub 34 by the table positioner 38 in each of first and second directions within a reference plane that at least generally coincides with a supporting surface 33 of the table 32. Double-headed arrow 50a in Figure 1 represents one direction in which the table 32 may be moved relative to the table tub 34 within this reference plane, and which may define a longitudinal dimension or axis (e.g., coinciding with or defining the long axis of the supporting surface 33 of the table 32). The table 32 may also be moved relative to the table tub 34 in a direction that is orthogonal to the view presented in Figure 1, and which may define a lateral dimension (e.g., see Figure 2, which includes one double-headed arrow 50a to define the noted longitudinal dimension or axis, and which includes another double-headed arrow 50b to define a lateral dimension or axis). A patient would typically lie head-to-toe in the longitudinal dimension (e.g., coinciding with double-headed arrow 50a) on the supporting surface 33 of the table 32. If the patient were lying on his/her back in this fashion, the patient's shoulders would be spaced in the lateral dimension (e.g., coinciding with double-headed arrow 50b).

The table positioner 38 may provide multiple movements or movement types for the table 32. The table positioner 38 may be configured to move the table 32 relative to the table tub 34 in the above-noted manner (e.g., in each of the longitudinal and lateral dimensions coinciding with double-headed arrows 50a, 50b, respectively). The table positioner 38 may be configured to collectively move the table 32 and the table tub 34 in the vertical dimension, and as indicated by the double-headed arrow 54 (e.g., up and down relative to a floor 66, which may support one or more components of the medical imaging system 10). The table positioner 38 may be configured to collectively move the table 32 and the table tub 34 at least generally about an axis 46 that extends in the lateral dimension, that is horizontally disposed, or both, and as indicated by the double-headed arrow 52. This type of motion may be characterized as changing an angle between horizontal and the longitudinal dimension or axis 50a of the supporting surface 33 of the table 32. Another characterization of this motion is that it is a "tilting" of the table 32, for instance a "longitudinal tilting " of the table 32 (e.g., raising the head and simultaneously lowering the feet of the patient; lowering the head and simultaneously raising the feet of the patient). Therefore, the axis 46 may be referred to as a "tilt axis 46." The tilt axis 46 may be disposed at any appropriate location in the vertical dimension (e.g., double-headed arrow 54) and at any appropriate location in the longitudinal dimension (e.g., double-headed arrow 50a) of the table 32.

The table positioner 38 may be of any appropriate size, shape, configuration, and/or type to move the table 32 in any desired manner. In the illustrated embodiment, the table positioner 38 includes a base 40 that is disposed on the floor 66. The table positioner 38 utilizes a column 42 (e.g., the shaft of an appropriate cylinder) that may be both extended and retracted to raise and lower, respectively, the table 32 in the vertical dimension (e.g., to move the table 32 along an axis corresponding with the double-headed arrow 54). A joint 44 of any appropriate type allows the table positioner 38 to move the table 32 at least generally about the tilt axis 46. Part of the table positioner 38 (not shown) may be located within the table tub 34 or otherwise to move the table 32 relative to the table tub 34 in the above-noted longitudinal and lateral dimensions (e.g., in accordance with the two double-headed arrows 50a-b shown in Figure 2).

The medical imaging system 10 of Figure 1 includes a foot-activated controller 70 for controlling one or more aspects of the operation of at least one of, and including both of, the imaging assembly 12 and the table positioner 38. Therefore, the foot-activated controller 70 may be referred to as a multi-function controller. In any case, any appropriate communication link 100a may exist between the foot-activated controller 70 and the table positioner 38. Similarly, any appropriate communication link 100b may exist between the foot-activated controller 70 and the imaging assembly 12. The communication links 100a, 100b may be of a common or different type. In one embodiment, each communication link 100a, 100b is a wireless communication link.

A more detailed view of the medical imaging system 10 is presented in Figure 2. Here the imaging assembly 12 includes camera equipment 14 (e.g., for acquiring an x-ray image, for acquiring a tomography image, for acquiring a fluoroscopy image, for acquiring an endoscopic image, and any combination thereof), a support arm 16 for the camera equipment 14, and one or more monitors 18 (two shown) for displaying an acquired image. The lower portion of the table tub 34 is attached to a pedestal 36 in the Figure 2 configuration. The table positioner 38 is not shown in Figure 2, but is able to move the table 32 relative to the table tub 34 in each of the longitudinal and lateral dimensions (double-headed arrows 50a-b), is able to collectively move the table 32 and table tub 34 in the vertical dimension (double-headed arrow 54), and is able to collectively and longitudinally tilt the table 32 and table tub 34 at least generally about the tilt axis 46 (double-headed arrow 52).

The foot-activated controller 70 is operatively interconnected with each of the table positioner 38 and the imaging assembly 12 by a communication link 100. In accordance with the foregoing, the communication link 100 may be of any appropriate type (e.g., wireless). A separate communication link 100 may be provided between the foot-activated controller 70 and each of the table positioner 38 and the imaging assembly 12 or otherwise. The medical imaging system 10 may also include one or more hand-activated controllers 62, where each such hand-activated controller 62 is operatively interconnected with at least one of the table positioner 38 and the imaging assembly 12 by a communication link 64. Each such communication link 64 may be of any appropriate type (e.g., wireless). A separate communication link 64 may be provided between any particular hand-activated controller 62 and each of the table positioner 38 and the imaging assembly 12 or otherwise. A separate hand-activated controller 62 could also be provided for each of the table positioner 38 and the imaging assembly 12 (not shown).

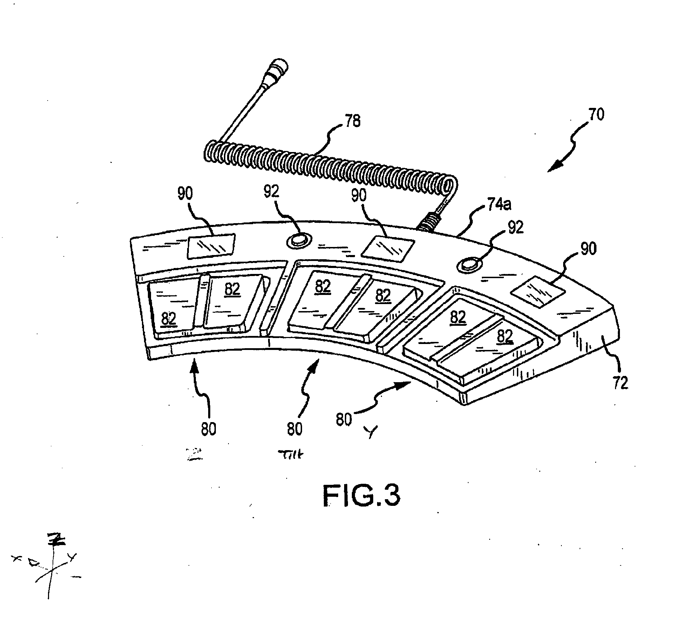

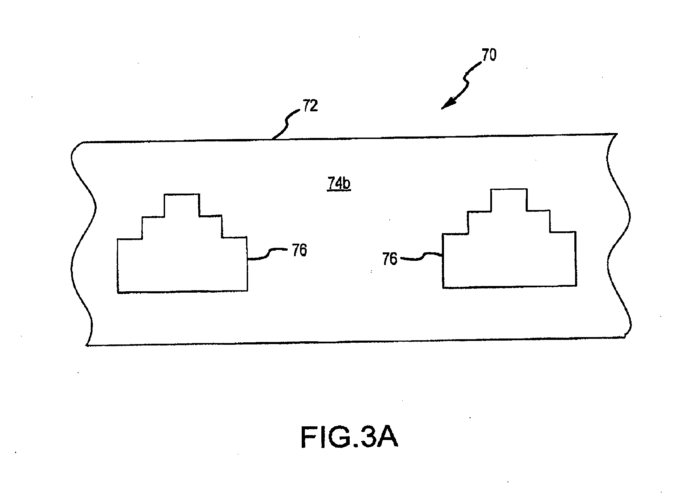

One embodiment of the foot-activated controller 70 is illustrated in more detail in Figure 3. The foot-activated controller 70 includes a housing or base 72 which may be disposed upon the floor 66, which may be of any appropriate size, shape, and/or configuration, and which may be formed from any appropriate material or combination of materials. A surface 74a of the housing 72 incorporates at least one group 80 of pedals or actuators 82. Any appropriate number of pedal groups 80 may be utilized by the foot-activated controller 70. Each pedal group includes 80 at least one pedal or actuator 82. Each pedal group 80 may include any appropriate number of pedals 82, including were each pedal group 80 includes the same number of pedals 82, as well as where at least one pedal group 80 utilizes a different number of pedals 82 that at least one other pedal group 80. In the illustrated embodiment, there are three pedal groups 80, and each pedal group 80 includes two pedals 82.

The individual pedals 82 may be of any appropriate size, shape, configuration, and/or type. In the illustrated embodiment, each pedal group 80 is in the form of a left/right rocker switch. Other "switch" configurations may be appropriate for each pedal 82. Each pedal 82 may be of the same "switch configuration" or otherwise. Any appropriate function or combination of functions may be initiated by activating a particular pedal 82.

In one embodiment, each of the pedals 82 in the same pedal group 80 provides at least somewhat of a related function. Consider the case where the foot-activated controller 70 is being used to control the motion of the table 32 for the medical imaging system of Figures 1-2. One pedal group 80 may be utilized to control the position of the table 32 in the vertical dimension and coinciding with the double-headed arrow 54 in Figures 1 and 2 (e.g., one pedal 82 in this pedal group 80 being used to raise the table 32, and the other pedal 82 in this pedal group 80 being used to lower the table 32). One pedal group 80 may be utilized to control the tilt angle of the table 32 and coinciding with the double-headed arrow 52 in Figures 1 and 2 (e.g., one pedal 82 in this pedal group 80 being used to raise the patient's head and simultaneously lower the patient's feet (e.g., move the table 32 at least generally about the tilt axis 46 in one direction), and the other pedal 82 in this pedal group 80 being used to lower the patient's head and simultaneously raise the patient's feet (e.g., move the table 32 at least generally about the tilt axis 46 in the opposite direction)). One pedal group 80 may be utilized to control the position of the table 32 in the lateral dimension and coinciding with the double-headed arrow 50b in Figure 2 (e.g., one pedal 80 in this pedal group 82 being used to move the table 32 at least generally away from the camera equipment 14 in the lateral dimension, and the other pedal 82 in this panel group 80 being used to move the table 32 at least generally toward the camera equipment 14 in the lateral dimension).

The upper surface 74a also incorporates a controller display 90 for each pedal group 80. Each controller display 90 may be of any appropriate size, shape, configuration, and/or type (e.g., a liquid crystal display or LCD). Generally, the function of at least one pedal 82 may be presented on the corresponding controller display 90 in a manner that will be discussed in more detail below. In one embodiment, the function of each pedal 82 in each pedal group 80 is simultaneously presented on the corresponding controller display 90 at a given time. In one embodiment, the function of a single pedal 82 is presented on its corresponding controller display 90 at a given time. Since each pedal group 80 could conceivably include a single pedal 82, the foot-activated controller 70 could provide a controller display 90 for each pedal 82. However and for the case where there are multiple pedals 82 that each provide at least somewhat of a common function (e.g., changing the position of the table 32 in the vertical dimension), it may be beneficial to include these pedals 82 in a common pedal group 80 and to utilize a single controller display 90 for this particular pedal group 80.

One or more additional switches 92 may be incorporated on the upper surface 74a of the foot-activated controller 70. Any appropriate number of switches 92 may be utilized, and each individual switch 92 may be disposed at any appropriate location. Each switch 92 may provide any appropriate function or combination of functions (e.g., turning on/off an image saving function; turning on/off room lights; brightening/dimming room lighting; turning on/off a power injector).

The foot-activated controller 70 may communicate in any appropriate manner with one or more medical devices (e.g., the imaging assembly 12 and/or table positioner 38 of the medical imaging system 10 of Figures 1-2), including without limitation wirelessly or via appropriate cabling, wiring, or the like. Figure 3 illustrates a communication cable 78 that may be operatively interconnected with the foot-activated controller 70 and one or more medical devices. In this regard and referring now to Figure 3A, a rear surface 74b of the housing or base 72 may include one or more communication ports 76. Each communication port 76 may be of any appropriate type (e.g., wireless, serial) and allows the foot-activated controller 70 to communicate with any appropriate device. One or more different types of communication ports 76 may be provided for the foot-activated controller 70, and each communication port 76 may be disposed at any appropriate location on the housing 72 of the foot-activated controller 70.

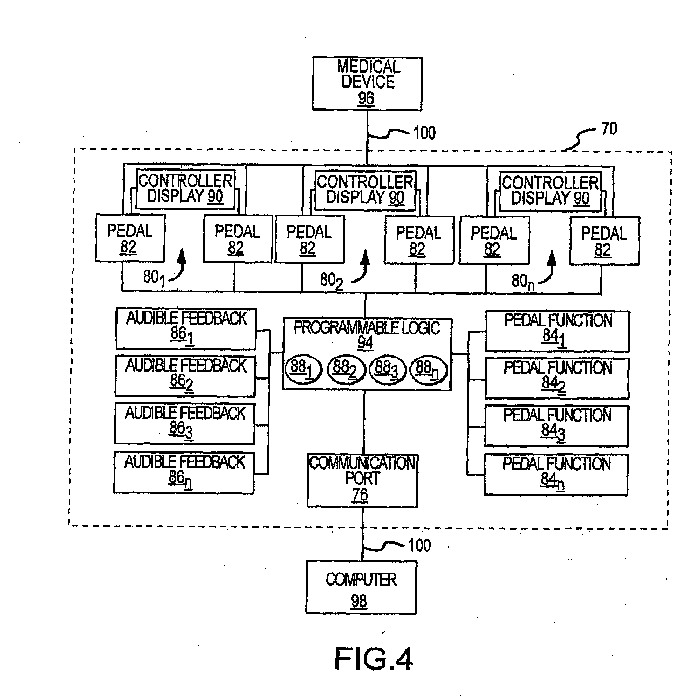

Figure 4 presents a representative functional schematic that may be utilized by the foot-activated controller 70, and for the case where the foot-activated controller 70 is operatively interconnected with a medical device 96 (e.g., table positioner 38; imaging assembly 12) via an appropriate communication link 100 of any appropriate type (e.g., wireless, serial cable). The foot-activated controller 70 includes a programmable logic 94 which may be of any appropriate configuration. Generally, the logic 94 may be programmed using an external or remote computer 98 of any appropriate type (e.g., a laptop) via a communication link 100 of any appropriate type (e.g., wireless, serial cable), along with a communication port 76 of the foot-activated controller 70 that is operatively interconnected with the programmable logic 94. Each communication port 76 of the foot-activated controller 70 may communicate with its programmable logic 94 in any appropriate manner.

The various pedal groups 80 of the foot-activated controller 70 may be operatively interconnected with the programmable logic 94 in any appropriate manner. More generally, each of the various pedals 82 may be operatively interconnected with the programmable logic 94 in any appropriate manner. Any appropriate programming may be undertaken in relation to each pedal 82. Although each pedal 82 may be programmed, each of the pedals 82 may not be required for a given application/procedure, and therefore programming of any such unused pedals 82 may not be undertaken in each instance.

One or more pedal functions 84 may be stored in any appropriate manner and used to configure the programmable logic 94 of the foot-activated controller 70 of Figure 4. Any appropriate number of pedal functions 84 may be made available for assignment to each particular pedal 82. Generally, a pedal function 84 initiates a certain action upon its execution (e.g., activation of a pedal 82 having this assigned pedal function 84).

One or more audible feedbacks 86 may be stored in any appropriate manner and used to configure the programmable logic 94 of the foot-activated controller 70 of Figure 4. Any appropriate number of audible feedbacks 86 may be made available for assignment to each particular pedal 82. Each audible feedback 86 differs in at least some respect from the other audible feedbacks 86. Each audible feedback 86 may be of any appropriate type, for instance in the form of a tone, a pulsed tone, a voice message, a melody, or the like. Assigning a different audible feedback 86 to each pedal 82 may be used to identify each particular pedal 82 during use of the foot-activated controller 70.

Multiple pedal profiles 88 may be stored in relation to the foot-activated controller 70 of Figure 4. Each pedal profile 88 includes an assigned pedal function 84 and assigned audible feedback 86 for each pedal 82 that is to be used by the foot-activated controller 70 for a particular application/procedure. Any appropriate number of pedal profiles 88 may be stored, and may be accessed by personnel in any appropriate manner (e.g., through one of the switches 92 on the foot-activated controller 70).

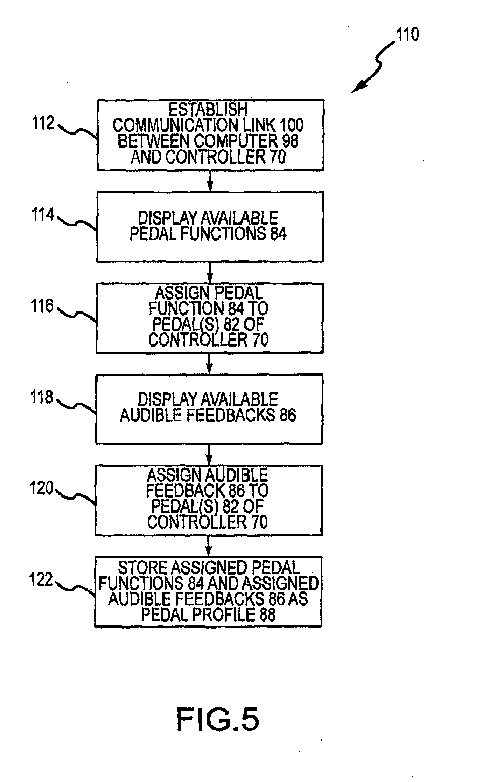

One embodiment of a protocol for programming the foot-activated controller of Figures 3-4 is illustrated in Figure 5 and is identified by a reference numeral 110. The programming protocol 110 includes establishing a communication link 100 between an external or remote computer 98 and the foot-activated controller 70 (e.g., via an appropriate communication port 76 on the foot-activated controller 70). One or more pedal functions 84 may be displayed (e.g., on the computer 98) in any appropriate manner through execution of step 114. In one embodiment, a listing of all pedal functions 84 that are available for assignment to the pedals 82 may be presented on an appropriate display (e.g., via a drop-down menu). A pedal function 84 may be assigned to one or more of the pedals 82 of the foot-activated controller 70 (including each of the pedals 82) through execution of step 116.

One or more audible feedbacks 86 may be displayed (e.g., on the computer 98) in any appropriate manner through execution of step 118 of the programming protocol 110 of Figure 5. In one embodiment, a listing of all audible feedbacks 86 that are available for assignment to the pedals 82 of the foot-activated controller 70 may be presented on an appropriate display (e.g., via a drop-down menu). An audible feedback 86 may be assigned to one or more of the pedals 82 of the foot-activated controller 70 (including each of the pedals 82) through execution of step 120. The assigned pedal functions 84 (step 116) and assigned audible feedbacks 86 (step 120) may be stored as a pedal profile 88 through execution of step 122. It should be appreciated that the assignment of the various function to a particular pedal 82 may be undertaken in any appropriate order.

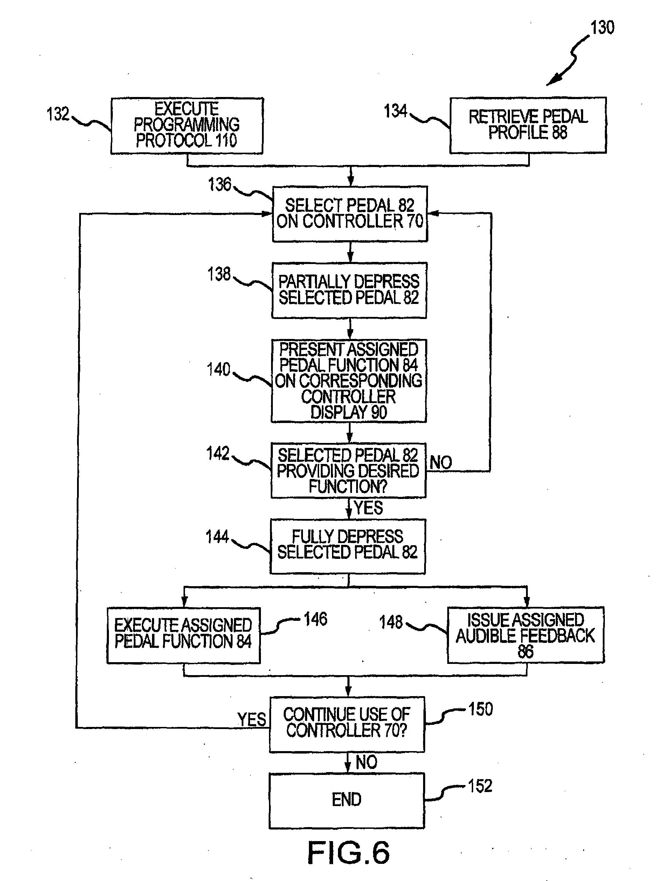

The foot-activated controller 70 of Figures 3-4 may be operated in accordance with an operations protocol 130 that is presented in Figure 6. Other protocols may be appropriate. The operations protocol 130 accommodates executing the programming protocol 110 of Figure 5 (step 132), as well as retrieving a stored pedal profile 88 (step 134). Steps 132 and 134 are each generally directed to the programmability for the pedals 82 of the foot-activated controller 70, although such may not be required in all instances. Once the desired pedal assignments have been realized in any appropriate manner, the foot-activated controller 70 may be used to control one or more aspects of the operation of at least one medical device 96 (Figure 4).

Step 136 of the operations protocol 130 of Figure 6 is directed to selecting a pedal 82 for initiating the execution of a desired function. The operations protocol 130 is configured to provide operator feedback before the function is actually initiated. In this regard, step 138 is directed to partially depressing or "tapping" the selected pedal 82 (e.g., moving the selected pedal 82 from an inactive position to an intermediate position, and at least generally in a first direction). Once the selected pedal 82 is partially depressed, the assigned pedal function is presented on the corresponding controller display 90 through execution of step 140 of the operations protocol 130. This functionality may be displayed at one or more locations and in any appropriate manner (e.g., graphically, pictorially, or any combination thereof). The functionality may be conveyed in any appropriate manner, including without limitation using one or more still images, using one or more moving images, using a single color, using multiple colors, or any combination thereof. In any case, this provides a visual feedback to the operator of the foot-activated controller 70. The audible feedback 86 that is assigned to the partially depressed pedal 82 may also be issued at this time (not shown in Figure 6, but from partially depressing or "tapping" a pedal 82). Therefore, the operations protocol 130 may be configured to provide multiple operator feedbacks regarding each pedal 82 of the foot-activated controller 70 before the assigned pedal function 84 is actually initiated.

In the event that the operator has inadvertently selected the wrong pedal 82 of the foot-activated controller 70, the operations protocol 130 of Figure 6 allows another pedal 82 to be selected in the above-noted manner and without initiating its assigned pedal function 84 (e.g., step 142). Otherwise, the protocol 130 proceeds to step 144, where the selected pedal 82 may be fully depressed or activated (e.g., by a movement of the selected pedal 82 to its actuating position and at least generally in the first direction - a movement of a pedal 82 from its inactive position to its actuating position will thereby pass through the noted intermediate position). Actuation of the selected pedal 82 may initiate one or more actions. Step 146 of the operations protocol 130 indicates that the assigned pedal function 84 may be executed. Step 148 of the operations protocol 130 indicates that the assigned audible feedback 148 may be issued.

Various procedures may of course require multiple actuations of one or more of the pedals 82 of the foot-activated controller 70. The operations protocol 130 accommodates for such scenarios through execution of step 150 and a return to step 136 for repetition in accordance with the foregoing. Otherwise, the operations protocol 130 may be terminated in any appropriate manner through execution of step 152.

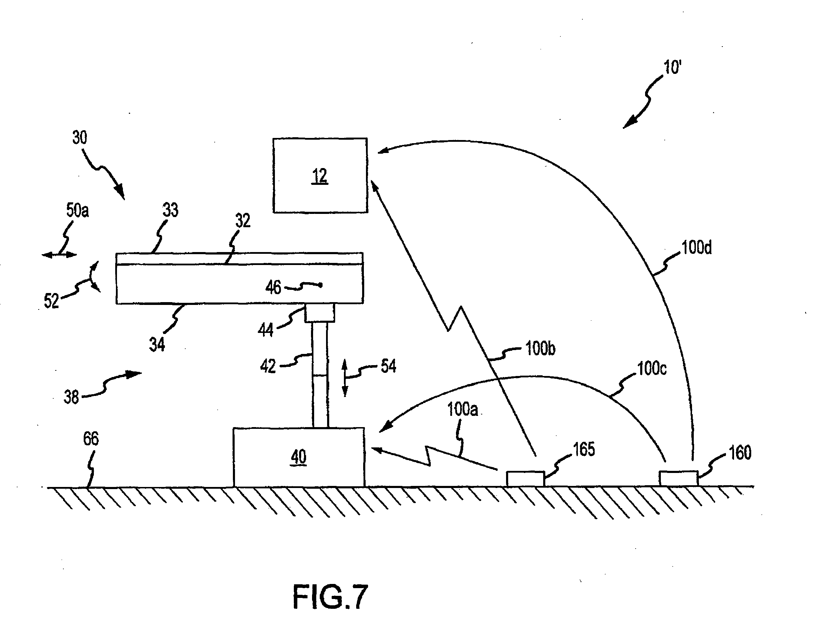

A variation of the medical imaging 10 of Figure 1 is illustrated in Figure 7 and is identified by a reference numeral 10'. Corresponding components of these imaging systems 10, 10' are identified by the same reference numeral. The medical imaging system 10' includes a foot-activated controller 160 and a foot-activated controller 165, each of which may be operatively interconnected with at least one of the imaging assembly 12 and the table assembly 30 in any appropriate manner (e.g., by a communication link 100 of any appropriate type). More specifically, the foot-activated controller 160 and the foot-activated controller 165 each may be operatively interconnected with at least the imaging assembly 12 (e.g., one or both of the foot-activated controller 160 and the foot-activated controller 165 may, but are not required to be, also be operatively interconnected with the table assembly 30, at least one other medical device, or both), each may be operatively interconnected with at least the table assembly 30 (e.g., one or both of the foot-activated controller 160 and the foot-activated controller 165 may, but are not required to be, also be operatively interconnected with the imaging assembly 12, at least one other medical device, or both), or both. That is, the foot-activated controllers 160, 165 communicate with at least one common medical device, although one or both of the foot-activated controllers 160, 165 may communicate with one or more additional medical devices as desired/required. The foot activated controllers 160, 165 could communicate with two or more common medical devices.

The foot-activated controller 165 and the foot-activated controller 160 may communicate with one or more common medical devices (e.g., imaging assembly 12; table assembly 30), and the foot-activated controller 160 may control a larger number of functions than the foot-activated controller 165 in relation to each medical device in communication with each of the foot-activated controllers 160, 165. In one embodiment, the foot-activated controllers 160, 165 each communicate with each of the imaging assembly 12 and table assembly 30. In another embodiment, one of the foot-activated controllers 160, 165 communicates with only one of the imaging assembly 12 and the table assembly 30, while the other of the foot-activated controllers 160, 165 communicates with each of the imaging assembly 12 and table assembly 30. In another embodiment, each of the foot-activated controllers 160, 165 each communicate with a common one of the imaging assembly 12 and the table assembly 30, but not the other of the imaging assembly 12 and the table assembly 30.

A number of characterizations may be made in relation to the foot-activated controllers 160, 165 of the medical imaging system 10'. In one embodiment, the foot-activated controller 160 may be configured as a full-function controller for at least one medical device (e.g., the imaging assembly 12, the table assembly 30), while the foot-activated controller 165 may be configured as a limited-function controller for at least this same medical device (e.g., the imaging assembly 12, the table assembly 30). In another embodiment, the foot-activated controller 165 may be configured to provide a first set of functions in relation to at least one medical device (e.g., the imaging assembly 12, the table assembly 30), and the foot-activated controller 160 may be configured to provide a second set of functions in relation to at least this same medical device (e.g., the imaging assembly 12, the table assembly 30), where each function in the first set of functions (foot-activated controller 165) is also a function in the second set of functions (foot-activated controller 160), and where the number of functions in the second set of functions (foot-activated controller 160) is greater than the number of functions in the first set of functions (foot-activated controller 165). In yet another embodiment, the foot-activated controller 165 may be configured to provide a first number of functions in relation to at least one medical device (e.g., the imaging assembly 12, the table assembly 30), and the foot-activated controller 160 may be configured to provide a second number of functions in relation to at least this same medical device (e.g., the imaging assembly 12, the table assembly 30), where the second number of functions (foot-activated controller 160) is greater than the first number of functions (foot-activated controller 165), and including without limitation where each function provided by the foot-activated controller 165 is also available through the foot-activated controller 160.

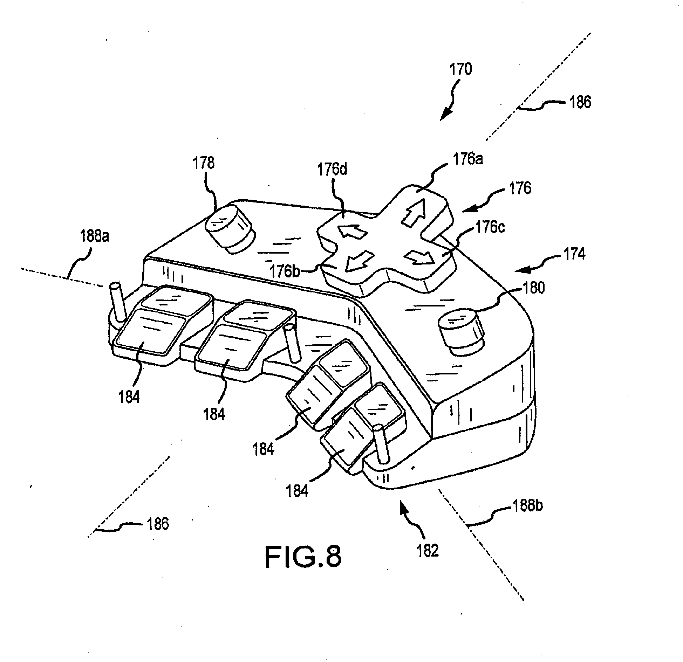

Each of the foot-activated controller 160 and the foot-activated controller 165 may be of any appropriate size, shape, configuration, and/or type. The foot-activated controller 160 could be of the type disclosed in commonly assigned U.S. Patent No. 5,883,615. In one embodiment, the foot-activated controller 165 is in the form of the foot-activated controller 70 shown in Figure 3. That is, the foot-activated controller 70 could be programmed such that it provided less functionality than the foot-activated controller 160 in relation to at least one common medical device. Another embodiment of a controller is illustrated in Figure 8, is identified by reference numeral 170, and may be used as the foot-activated controller 165 in the medical imaging system 10' of Figure 7.

The foot-activated controller 170 of Figure 8 includes a housing or base 172 which may be disposed upon the floor, which may be of any appropriate size, shape, and/or configuration, and which may be formed from any appropriate material or combination of materials. This base 172 is subject to a number of characterizations. One is that the base 172 is of an at least generally V-shaped configuration in a top view, where the open end of the "V" defines a front or forward end of the foot-activated controller 170. Another is that the base 172 is of an at least generally boomerang-shaped configuration in a top view where an opening defined by this configuration defines a front or forward end of the foot-activated controller 172. In each of these characterizations, a plurality of first actuators 184 may be characterized as being in the corresponding "open end".

The plurality of first actuators 184 in the case of the foot-activated controller 170 of Figure 8 are incorporated on a lower level 182 of the base 172, and are disposed at a common elevation. The individual actuators 184 may be of any appropriate size, shape, configuration, and/or type. In the illustrated embodiment, each actuator 184 is in the form of a pedal actuator. Each actuator 184 may be of the same configuration, although such may not be required in each instance. Any appropriate function or combination of functions may be initiated by activating a particular actuator 184. In one embodiment, each actuator 184 provides a separate imaging-related or image acquisition function (e.g., to acquire a digital fluoroscopy image; to acquire a super digital fluoroscopy image; to turn on a rad/spot for a full x-ray image (the term "rad" being short for "radiograph", and the term "spot" being short for "digital spot"; each refers to a high-dose x-ray exposure that has diagnostic clarity, although the term "rad" is more typically associated with film and the term "spot" is more typically associated with digital images); to control a magnification mode for an x-ray zoom).

Any appropriate number of first actuators 184 may be incorporated on the lower level 182 of the foot-activated controller 170 of Figure 8. Four first actuators 184 are utilized by the illustrated embodiment. The manner in which the various first actuators 184 may be arranged on the lower level 182 is subject to a number of characterizations. The foot-activated controller 170 may be characterized as being bisected in a lateral dimension by a central, longitudinal axis 186. This lateral dimension coincides with a width of the controller 170. In one embodiment, an operator's foot would be advanced at least generally orthogonally to the lateral dimension to access the controller 170. In one embodiment, the controller 170 would be positioned such that the first actuators 184 at least generally project in the direction of an operator. In one embodiment, the controller 170 would be positioned such that the first actuators 184 define the forward or leading end of the controller 170.

Each first actuator 184 that is disposed on a first side of the above-noted central, longitudinal axis 186 (e.g., the "left side" of the axis 186 in the view presented in Figure 8) may be disposed in a common first orientation, and each first actuator 184 that is disposed on a second side of the central, longitudinal axis 186 (e.g., the "right side" of the axis 186 in the view presented in Figure 8) may be disposed in a common second orientation, where at least one first actuator 184 is disposed on the noted first side of the axis 186, and where at least one first actuator 184 is disposed on the noted second side of the axis 186. In the illustrated embodiment, two first actuators 184 are disposed on one side of the central, longitudinal axis 186, and two first actuators 184 are disposed on the opposite side of the central, longitudinal axis 186. A common number of first actuators 184 may be disposed on each side of the central, longitudinal axis 186, including without limitation where the arrangement of all first actuators 184 disposed on the noted first side of the axis 186 are the mirror image of the arrangement of all first actuators 184 disposed on the noted second side of the axis 186.

Each first actuator 184 that is disposed on a common side of the central, longitudinal axis 186 may move at least generally about a common axis 188a or 188b. In the illustrated embodiment, the first actuators 184 that are disposed on the first side of the central, longitudinal axis 186 each move at least generally about an axis 188a (e.g., the "left side" of the axis 186 in the view presented in Figure 8), while the first actuators 184 that are disposed on the second side of the central, longitudinal axis 186 each move at least generally about an axis 188b (e.g., the "right side" of the axis 186 in the view presented in Figure 8). The included angle between these axes 188a, 188b may be obtuse (e.g., between 90° and 180°). In one embodiment, the included angle between the axes 188a, 188b is within a range from about 135° to about 175°, inclusive. The axes 188a, 188b may also define how the housing 172 extends in the lateral dimension.

At least one second actuator 176 may be incorporated on an upper level 174 of the base 172. In the illustrated embodiment, a single second actuator 176 is centrally disposed in the lateral dimension on the upper level 174 (e.g., located on the central, longitudinal axis 186). Each such second actuator 176 may be of any appropriate size, shape, configuration, and/or type. In the illustrated embodiment, a single second actuator 176 in the form of a four-position switch is utilized. Other configurations may be appropriate.

Any appropriate function or combination of functions may be initiated by activating any particular second actuator 176. In one embodiment, each of the individual sections 176a-d (e.g., in effect four different actuators) controls a particular movement of the table 32 (e.g., section 176a may move the table 32 in an upward direction along an axis coinciding with double-headed arrow 54 in Figure 7; section 176b may move the table 32 in a downward direction along an axis coinciding with double-headed arrow 54 in Figure 7; section 176d may move the table 32 in one direction along an axis coinciding with double-headed arrow 50a in Figure 7; section 176d may move the table 32 in the opposite direction along an axis coinciding with double-headed arrow 50a in Figure 7).

The upper level 174 of the foot-activated controller 170 may incorporate a mode switch 178, which may be of any appropriate size, shape, configuration, and/or type. Activation of the mode switch 178 may change the structure or the combination of structures that are moved by an actuation of the second actuator 176. For instance, one mode may configure the second actuator 176 to move the table 32, while another mode may configure the second actuator 176 to move part of the imaging assembly 12 (e.g., an imaging chain). Any appropriate number of modes could be accessed through activation of the mode switch 178.

The upper level 174 of the foot-activated controller 170 may incorporate a save image switch 180. Activation of the save-image switch 180 may initiation the saving of an acquired image at one or more appropriate locations. Although each of the mode switch 178 and the save image switch 180 may be disposed at any appropriate location, in one embodiment they are positioned in the mirror image of each other relative to the central, longitudinal axis 186.

The foot-activated controller 170 of Figure 8 could be configured incorporate one or more of the various features discussed above in relation to the foot-activated controller 70 of Figure 3, individually and in any combination (e.g., programmability; multiple controller displays 90, for instance one for each pedal 186; providing function information prior to actuation of an actuator; using multiple feedbacks; providing different audible feedbacks for different functions; storing multiple actuator profiles). Each of the features of the foot-activated controller 70 could be utilized by the foot-activated controller 170. The foot-activated controller 70 could also be used as the foot-activated controller 165 in the medical imaging system 10' of Figure 8.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, may fall within the scope of the appended claims. The embodiments described hereinabove are further intended to explain best modes known of practicing the invention and to enable others skilled in the art to utilize the invention in such embodiments or in other embodiments and with various modifications required by the particular application(s) or use(s) of the present invention. It is intended that the appended claims be construed to include alternative embodiments to the extent permitted by law.

## Claims

1. A medical system, comprising:
a first medical device (12, 30);
a first controller (170) operatively interconnected with said first medical device and that is foot-activated, wherein said first controller comprises a plurality of first actuators (184) that are disposed at a common elevation and that each communicate with said first medical device, wherein each said first actuator (184) comprises a pedal actuator, wherein a central, longitudinal axis (186) bisects said first controller in a lateral dimension, wherein each said first actuator disposed on a first side of said central, longitudinal axis is disposed in a common first orientation, wherein at least one said first actuator is disposed on said first side of said central, longitudinal axis, wherein each said first actuator disposed on a second side of said central, longitudinal axis is disposed in a common second orientation, wherein at least one said first actuator is disposed on said second side of said central, longitudinal axis, and wherein said first and second orientations are different; and
a second controller (176) operatively interconnected with said first medical device and that is foot-activated **characterized in that** said system further comprises:
a second medical device (12, 30), wherein said first controller (170) is operatively interconnected with said second medical device, and wherein there is a lack of a communication link between said second controller (176) and said second medical device.

2. The medical system of Claim 1, wherein said first medical device (12, 30) is selected from the group consisting of imaging equipment and a table assembly.

3. The medical system of Claim 1, wherein said first medical device comprises a table assembly (30) that comprises a movable table (32).

4. The medical system of Claim 1, wherein said first medical device comprises imaging equipment (12).

5. The medical system of Claim 1, wherein said first medical device is one of imaging equipment (12) and a table assembly (30), and wherein said second medical device is the other of said imaging equipment (12) and said table assembly (30).

6. The medical system of Claim 1, wherein at least two said first actuators (184) are disposed on said first side of said central, longitudinal axis (186), and wherein at least two said first actuators (184) are disposed on said second side of said central, longitudinal axis.

7. The medical system of Claim 1, wherein each said first actuator (184) disposed on said first side of said central, longitudinal axis is movable at least generally about a first reference axis (188a), wherein each said first actuator (184) disposed on said second side of said central, longitudinal axis is movable at least generally about a second reference axis (188b), and wherein there is an obtuse included angle between said first and second reference axes.

8. The medical system of Claim 1, wherein a common number of said first actuators (184) are disposed on said first and second sides of said central, longitudinal axis (186), and wherein an arrangement of all said first actuators on said first side of said central, longitudinal axis is a mirror image of an arrangement of all said first actuators on said second side of said central longitudinal axis.

9. The medical system of Claim 1, wherein said first controller comprises an upper section (174) and a lower section (182), wherein said lower section comprises said plurality of first actuators (184).

10. The medical system of Claim 9, wherein said upper section (174) comprises at least one second actuator.

11. The medical system of Claim 9 or claim 10, wherein said upper section comprises a four-position switch (176).

## Patentansprüche

1. Medizinisches System, umfassend:
eine erste medizinische Vorrichtung (12, 30);
einen ersten Controller (170), der operativ mit der ersten medizinischen Vorrichtung verbunden ist und der fuß-aktiviert ist, wobei der erste Controller eine Vielzahl an ersten Aktoren (184) umfasst, die auf einer gemeinsamen Erhebung angeordnet sind und die jeweils mit der ersten medizinischen Vorrichtung kommunizieren, wobei jeder erste Aktor (184) einen Pedal-Aktor umfasst, wobei eine Mittellängsachse (186) den ersten Controller in einer lateralen Abmessung halbiert, wobei jeder erste Aktor, der auf einer ersten Seite der Mittellängsachse angeordnet ist, in einer gemeinsamen ersten Ausrichtung angeordnet ist, wobei mindestens ein erster Aktor auf der ersten Seite der Mittellängsachse angeordnet ist, wobei jeder erste Aktor, der auf einer zweiten Seite der Mittelachse angeordnet ist, in einer gemeinsamen zweiten Ausrichtung angeordnet ist, wobei mindestens ein erster Aktor auf der zweiten Seite der Mittellängsachse angeordnet ist und wobei die ersten und zweiten Ausrichtungen unterschiedlich sind; und
einen zweiten Controller (176), der operativ mit der ersten medizinischen Vorrichtung verbunden ist und der fuß-aktiviert ist, dadurch charakterisiert, dass das System weiterhin umfasst:
eine zweite medizinische Vorrichtung (12, 30), wobei der erste Controller (170) operativ mit der zweiten medizinischen Vorrichtung verbunden ist, und wobei eine Kommunikationsverbindung zwischen dem zweiten Controller (176) und der zweiten medizinischen Vorrichtung fehlt.

2. Medizinisches System nach Anspruch 1, wobei die erste medizinische Vorrichtung (12, 30) aus der Gruppe bestehend aus Bildgebungsausrüstung und einem Tisch-Aufbau ausgewählt wird.

3. Medizinisches System nach Anspruch 1, wobei die erste medizinische Vorrichtung einen Tisch-Aufbau (30) umfasst, der einen beweglichen Tisch (32) umfasst.

4. Medizinisches System nach Anspruch 1, wobei die erste medizinische Vorrichtung Bildgebungsausrüstung (12) umfasst.

5. Medizinisches System nach Anspruch 1, wobei die erste medizinische Vorrichtung eine aus Bildgebungsausrüstung (12) und einem Tisch-Aufbau (30) ist, und wobei die zweite medizinische Vorrichtung die andere aus Bildgebungsausrüstung (12) und dem Tisch-Aufbau (30) ist.

6. Medizinisches System nach Anspruch 1, wobei mindestens zwei erste Aktoren (184) auf der ersten Seite der Mittellängsachse (186) angeordnet sind, und wobei mindestens zwei erste Aktoren (184) auf der zweiten Seite der Mittellängsachse angeordnet sind.

7. Medizinisches System nach Anspruch 1, wobei jeder erste Aktor (184), der auf der ersten Seite der Mittellängsachse angeordnet ist, generell mindestens um eine erste Referenzachse (188a) beweglich ist, wobei jeder erste Aktor (184), der auf der zweiten Seite der Mittellängsachse angeordnet ist, generell mindestens um eine zweite Referenzachse (188b) beweglich ist, und wobei dort ein stumpf eingeschlossener Winkel zwischen der ersten und der zweiten Referenzachse ist.

8. Medizinisches System nach Anspruch 1, wobei eine gemeinsame Anzahl an ersten Aktoren (184) auf den ersten und zweiten Seiten der Mittellängsachse (186) angeordnet sind, und wobei eine Anordnung von allen ersten Aktoren auf der ersten Seite der Mittellängsachse ein Spiegelbild einer Anordnung von allen ersten Aktoren auf der zweiten Seite der Mittellängsachse ist.

9. Medizinisches System nach Anspruch 1, wobei der erste Controller einen oberen Abschnitt (174) und einen unteren Abschnitt (182) umfasst, wobei der untere Abschnitt eine Vielzahl an ersten Aktoren (184) umfasst.

10. Medizinisches System nach Anspruch 9, wobei der obere Abschnitt (174) mindestens einen zweiten Aktor umfasst.

11. Medizinisches System nach Anspruch 9 oder Anspruch 10, wobei der obere Abschnitt einen Vierpositionsschalter (176) umfasst.

## Revendications

1. Système médical comprenant :
un premier dispositif médical (12, 30) ;
un premier contrôleur (170) opérationnellement interconnecté avec ledit premier dispositif médical et activé au pied, dans lequel ledit premier contrôleur comprend une pluralité de premiers actionneurs (184) qui sont disposés selon une élévation commune et qui communiquent chacun avec ledit premier dispositif médical, dans lequel chacun desdits premiers actionneurs (184) comprend un actionneur à pédale,
dans lequel un axe central, longitudinal (186) coupe ledit premier contrôleur dans une dimension latérale, dans lequel chacun desdits premiers actionneurs disposés sur un premier côté dudit axe central, longitudinal est disposé selon une première orientation commune, dans lequel au moins un desdits premiers actionneurs est disposé sur ledit premier côté dudit axe central, longitudinal, dans lequel chacun desdits premiers actionneurs disposés sur un deuxième côté dudit axe central,
longitudinal est disposé selon une deuxième orientation commune, dans lequel au moins un desdits premiers actionneurs est disposé sur ledit deuxième côté dudit axe central, longitudinal et dans lequel lesdites première et deuxième orientations sont différentes ; et
un deuxième contrôleur (176), opérationnellement interconnecté avec ledit premier dispositif médical et activé au pied, **caractérisé en ce que** ledit système comprend :
un deuxième dispositif médical (12, 30), dans lequel ledit premier contrôleur (170) est opérationnellement interconnecté avec ledit deuxième dispositif médical, et dans lequel il y a un manque de lien de communication entre ledit deuxième contrôleur (176) et ledit deuxième dispositif médical.

2. Système médical selon la revendication 1, dans lequel ledit premier dispositif médical (12, 30) est choisi dans le groupe constitué d'un équipement d'imagerie et d'un ensemble de table.

3. Système médical selon la revendication 1, dans lequel ledit premier dispositif médical comprend un ensemble de table (30) qui comprend une table mobile (32).

4. Système médical selon la revendication 1, dans lequel ledit premier dispositif médical comprend un équipement d'imagerie (12).

5. Système médical selon la revendication 1, dans lequel ledit premier dispositif médical est un élément parmi un équipement d'imagerie (12) et un ensemble de table (30), et dans lequel ledit deuxième dispositif médical est l'autre parmi ledit équipement d'imagerie (12) et ledit ensemble de table (30).

6. Système médical selon la revendication 1, dans lequel au moins deux desdits premiers actionneurs (184) sont disposés sur ledit premier côté dudit axe central, longitudinal (186), et dans lequel au moins deux desdits premiers actionneurs (184) sont disposés sur ledit deuxième côté dudit axe central, longitudinal.

7. Système médical selon la revendication 1, dans lequel chacun desdits premiers actionneurs (184) disposés sur ledit premier côté dudit axe central, longitudinal est mobile au moins généralement autour d'un premier axe de référence (188a), dans lequel chacun desdits premiers actionneurs (184) disposés sur ledit deuxième côté dudit axe central, longitudinal est mobile au moins généralement autour d'un deuxième axe de référence (188b), et dans lequel il y a un angle inclus obtus entre lesdits premier et deuxième axes de référence.

8. Système médical selon la revendication 1, dans lequel un nombre commun desdits premiers actionneurs (184) est disposé sur lesdits premier et deuxième côtés dudit axe central, longitudinal (186), et dans lequel un agencement de tous lesdits premiers actionneurs sur ledit premier côté dudit axe central, longitudinal est une image en miroir d'un agencement de tous lesdits premiers actionneurs sur ledit deuxième côté dudit axe central, longitudinal.

9. Système médical selon la revendication 1, dans lequel ledit premier contrôleur comprend une section supérieure (174) et une section inférieure (182), dans lequel ladite section inférieure comprend ladite pluralité de premiers actionneurs (184).

10. Système médical selon la revendication 9, dans lequel ladite section supérieure (174) comprend au moins un deuxième actionneur.

11. Système médical selon la revendication 9 ou 10, dans lequel ladite section supérieure comprend un commutateur à quatre positions (176).
